(19) Europäisches Patentamt
European Patent Office
Office européen des brevets

(11) Publication number: 0 210 850 B1

# (12) EUROPEAN PATENT SPECIFICATION

(45) Date of publication of patent specification: 23.10.91

(51) Int. Cl.5: C07C 69/653, A61K 6/08, C08F 120/30, //C07C43/225, C07C43/23

(21) Application number: 86305751.9

(22) Date of filing: 25.07.86

## (54) Acrylic diesters of bisphenol alkyl ether, polymers prepared therefrom, and composites based thereon for dental use.

(30) Priority: 26.07.85 IT 2173285

(43) Date of publication of application: 04.02.87 Bulletin 87/06

(45) Publication of the grant of the patent: 23.10.91 Bulletin 91/43

(84) Designated Contracting States: AT BE CH DE FR GB LI NL SE

(56) References cited:
DE-A- 2 727 194
GB-A- 1 267 564

(73) Proprietor: MONTEDIPE S.r.l.
16, Piazza della Repubblica
I-20124 Milan(IT)

(72) Inventor: Bastioli, Catia
23, via Rivolta
I-28100 Novara(IT)
Inventor: Romano, Giancarlo
64, via Oslavia
I-10153 Torino(IT)
Inventor: Mazzocchi, Romano
28, via Possessione
I-28067 Pernate Novara(IT)

(74) Representative: Lamb, John Baxter et al
MARKS & CLERK 57-60 Lincoln's Inn Fields
London WC2A 3LS(GB)

EP 0 210 850 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to acrylic diesters of bisphenol alkyl ether, to polymers (acrylic resins) prepared therefrom, and to composites based thereon for dental use, i.e. composites for dental prosthesis containing said acrylic diesters.

As is known, for the preparation of dental fillings, crowns, bridges and parts to be substituted, there are utilized, additionally to gold and to porcelain, also synthetic substances such as polymers prepared from unsaturated compounds of the olefinic type, which are easily polymerizable. These polymeric substances offer substantial advantages with respect to prostheses made of gold or of porcelain, as regards their appearance. These polymeric substances actually permit better imitation of the colour of the teeth of different persons.

In recent years, the polymeric substances which have become most widely used in dentistry for the manufacture of dental fillings, crowns, artificial teeth and repairing work are polymethacrylates. Such polymethacrylates are generally obtained by thermal, chemical of photochemical polymerization of methyl methacrylate, so as to obtain a satisfactory degree of polymerization.

More recently, other synthetic substances, such as polyamides, polycarbonates and, chiefly, a great number of esters of methacrylic acid, have been synthesized and tested for their utilization in the field of dentistry. However, all endeavours made to substitute methyl methacrylate with other derivatives of acrylic or methacrylic acid were not sufficiently successful, so that methyl methacrylate has remained the most used compound in the dentistry field.

As is known, for dental restorations, polymerization can be conducted only at room temperature or at human body temperature. The main drawback of this cold polymerization is that a minor part of methyl methacrylate remians non-polymerized and can gradually release outside the composite. For this reason, the fillers based on methyl methacrylate are utilized only in the case of devitalized teeth.

With a view to improving the mechanical properties and, in particular, the resistance to abrasion of the synthetic substances, a few difunctional esters of methacrylic acid have been prepared which give rise to tridimensionally crosslinked products. The use of a few of these difunctional esters in the manufacture of dental prosthesis or fillings is describedin U.S. Patent No. US-A-3,066,112 (Bowen).

The difunctional esters of methacrylic acid described in US-A-3,066,112 are prepared through the reaction of phenols, in particular bisphenol A, with glycidyl methacrylate, giving rise to the following compound:

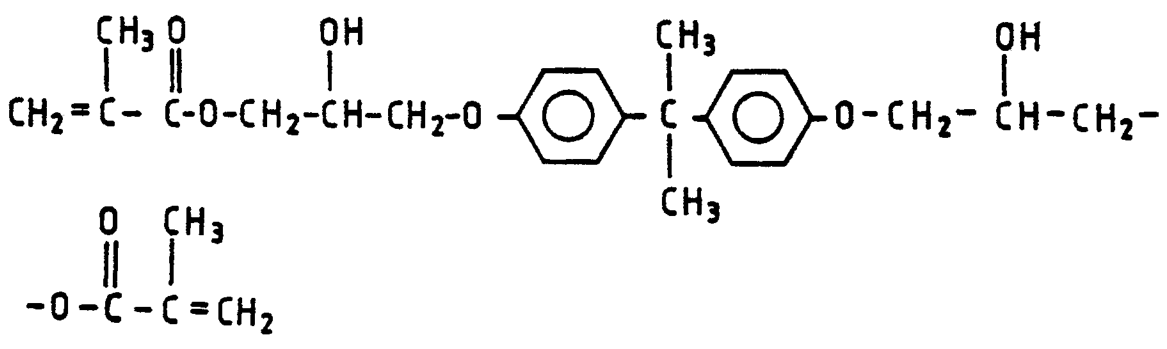

generally known as Bowen resin or resin BIS-GMA. The polymerization of such diester is started by an activator and by a catalyst, generally benzoyl peroxide, in the presence of diluents and of organic fillers.

The resin BIS-GMA, however, exhibits in practice various drawbacks which limit the use thereof. It exhibits, for example, a very high viscosity, of the order of 100 poises, with the consequent necessity of adding low molecular weight substances both to obtain high concentrations of filler in the composite and to achieve an acceptable degree of conversation of the resin during the polymerization.

In order to lower the viscosity of these resins there are added reactive diluents of the type of methyl methacrylate, of dimethacrylate glycols, such as ethylenglycol dimethacrylate and preferably triethylenglycol dimethacrylate or other suitable reactive extensors having a low molecular weight. The presence of these low molecular weight monomers entails various drawbacks such as a high shrinkage during the polymerization, the release of unreacted low molecular weight substances which are toxic for the dental pulp, plastification defects of the matrix and the like.

The BIS-GMA resins, furthermore, are not fully inert to moisture and, in the presence of water or saliva,

suffer from deterioration of the mechanical properties, colour changes due to degradation processes, microcavitation and plastification defects with consequent release of unreacted monomers.

GB 1 267 564 provides dental fillings and dental replacement parts having great stability to water and saliva prepared by polymerisation of a bifunctional monomer of general formula:

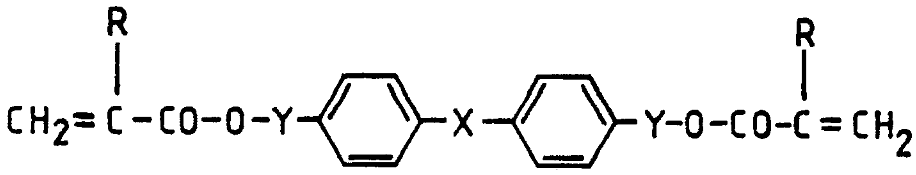

wherein R is a hydrogen atom or a methyl group, X is an alkylidene, including cycloalkylidene, or - $SO_2$ - group and Y is an oxyalkylene having two to five atoms, the oxygen atom of which is attached to the benzene ring, or substituted or unsubstituted alkylidene group having one to five carbon atoms and the benzene rings may optionally bear further substituents. We have now surprisingly found, according to the present invention, that composites for dental use, such as parts to be replaced, synthetic teeth, inner parts of a tooth, covering crowns, prosthesis articles and other dental preparations having higher resistance and stability properties as well as a low absorption of water, are obtainable when the monomer to be polymerized is a difunctional compound of an acrylic diester of a bisphenol alkyl ether of general formula:

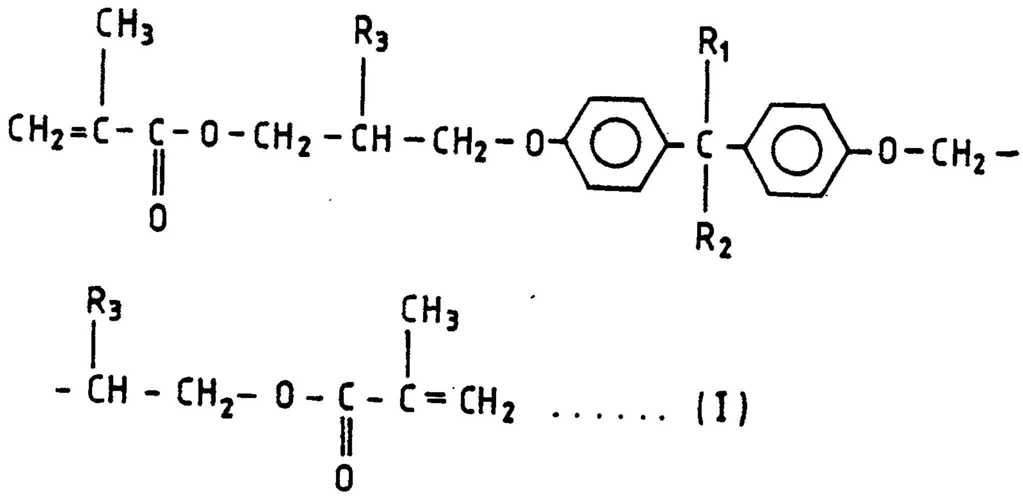

...... (I)

in which $R_1$ and $R_2$ may be, independently of each other, a linear or ramified alkyl radical containing from 1 to 7 carbon atoms with one or more hydrogen atoms substituted by a halogen such as fluorine, chlorine and bromine; and $R_3$ may be a hydrogen atom, a halogen atom, or a linear or ramified alkyl radical containing from 1 to 7 carbon atoms with, optionally, one or more hydrogen atoms substituted by a halogen such as fluorine, chlorine and bromine.

Accordingly, the present invention in one aspect provides an acrylic diester of a bisphenol alkyl ether as defined above.

According to a preferred embodiment of the present invention, $R_1$ and $R_2$ are perfluoro-alkyl radicals, and more preferably $-CF_3$, and $R_3$ is an alkyl radical, in particular a methyl radical.

One or more hydrogen atoms of the two benzenic rings of the acrylic diester of formula (I) can be substituted by alkyl or alkoxy radicals having a low number of carbon atoms, i.e. containing from 1 to 4 carbon atoms.

The acrylic diesters of bisphenol alkyl ether of formula (I) are generally low-viscosity liquids or relatively low-melting substances. They are preparable by the conventional methods of esterification or trans-esterification. For example, the diols of halogenated p,p'-dihydroxy-diphenyl-alkane can be directly esterified with acrylic or methacrylic acid in the presence of known esterification catalysts such as, for example, p.toluene-sulphonic acid. The preparation of the difunctional monomers of formula (I) can be also

conducted by trans-esterification of alkyl esters of acrylic or methacrylic acid, for example of methyl ester, with the diols mentioned hereinbefore, in the presence of an acid or a basic catalyst. Particularly advantageous is the preparation of the difunctional monomers of formula (I) by reaction of the diols with reactive derivatives of acrylic or methacrylic acid, for example chloride or anhydride.

The addition of a dehydrating agent may also prove advantageous. It is preferable to operate in an inert gas atmosphere, as well as to employ a polymerization inhibitor, such as for example 2,6-disubstituted phenol.

The diols of halogenated p,p'-dihydroxy-diphenyl-alkane are preparable by alkylation, with metallyl chloride, of halogenated bisphenol, in particular fluorinated bisphenol, and by subsequent hydroboration and oxidation of the obtained vinyl ether, according to the following scheme:

a)

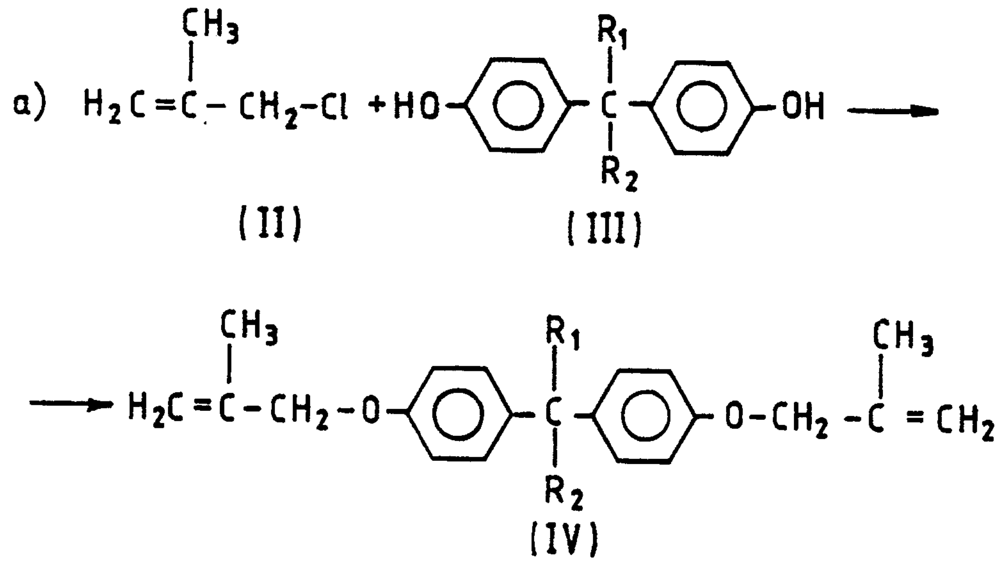

(II) (III)

(IV)

b)

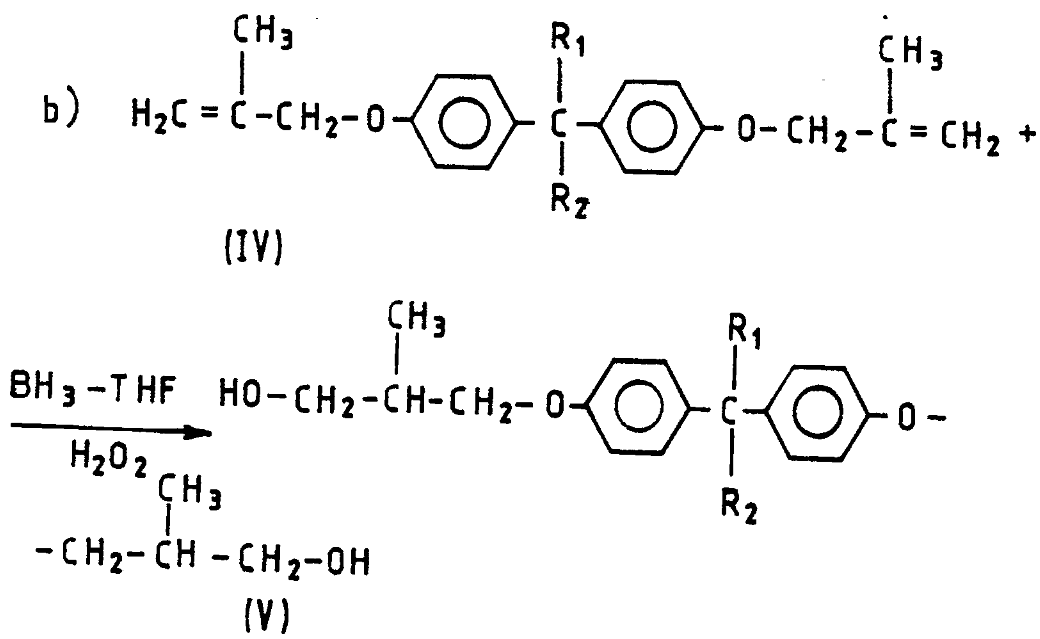


(IV)

(V)

The $BH_3$ -THF (tetrahydrofuran) complex is prepared 3 from $NaBH_4$ and $BF_3$ - $(C_2H_5)_2O$ in diglyme.
Oxidation is accomplished with $H_2O_2$ in the presence of NaOH.
The impure diol (V) can be purified by chromatography on a silica gel column.
The invention in another aspect provides an acrylic resin containing repeating units of an acrylic diester

of a bisphenol alkyl ether of formula (I).

The invention in a further aspect provides a dental composite comprising an acrylic diester of a bisphenol alkyl ether of formula (I), at least one inorganic filler, and a catalytic amount of a catalytic redox system.

The polymerization of the acrylic diester of bisphenol alkyl ether (I) is conducted in a conventional manner in the presence of substances capable of forming free radicals such as peroxides, nitriles of azocarboxylic acid, and redox catalysts. Utilizable peroxides are: benzoyl peroxide, lauryl peroxide, monoter.butyl-permaleate or ter.butyl-hydroperoxide. For the manufacture of substitution parts which are prepared separately, polymerization is carried out with lauryl peroxide by heating the mass placed in a mold, at a temperature ranging from 90 °C to 160 °C during a short period of time. It is preferable to conduct the polymerization at 120-160 °C in a hot air stream in order to achieve the highest possible cross-linking degree.

For the preparation of dental cements directly in the mouth there are utilized substances which are capable of acting as starters at room temperature or at the temperature of the human body, in particular redox systems. It is preferable to use substances which do not tend to change colour or to become dark. Suitable redox catalysts are benzoyl peroxide with N,N'-dimethyl-paratoluidine, hydroperoxides with thioureas, ethyl hydroperoxide with N,N-dimethyl-paratoluidine, benzyl ethers such as methyl benzyl ether with an ammonium activator, alphadiketones and methylamines, etc. The catalysts are preferably employed in a catalytic amount generally ranging from 0.05 to 5% by weight referred to the monomer.

Although an advantage of the dental composite and of the other dentistry products prepared according to the present invention resides in the possibility of not having to add any diluents, in a few particular cases such addition may be advantageous.

Suitable diluents are generally the esters of acrylic or methacrylic acid having a low molecular weight, such as ethylene glycol dimethacrylate; tri-ethyleneglycol dimethacrylate; tetra-ethyleneglycol dimethacrylate; bisphenol dimethacrylate; methyl methacrylate; and fluorinated diluents such as perfluoroalkyl methacrylates, alkylmethacrylates or alkyldimethacrylates.

Polymerization is generally conducted in the presence of fillers. Particularly advantageous fillers are quartzes, silicas, Al, Ba, Sr silicates and the like, zirconates, and aluminas, preferably those having a low surface area, with particle diameters below 40 $\mu$m, and treated with silanes of the type of methacryloxypropyl-silane, glass fibres, carbon fibres, etc., and submicronic inorganic fillers, brought to sizes of the order of 10-40 $\mu$m by means of a coating of methacrylic resin.

The amount of such fillers in the compounds may vary over a wide range, although concentrations in the range of from 30 to 86%, more preferably from 50 to 80% by weight, referred to the total weight of the composite, are generally used.

Another advantage of the present invention is that the diesters of formula (I) provide high degrees of conversion of the double methacrylic bonds in the polymerization; the low amounts of monomers, optionally non-polymerized, do not represent a problem for the dental pulp. In fact, said monomers posses a little mobility inside the matrix, which is polymerized, due to their high molecular weight.

Another advantage of the dental filler or of the part prepared from the polymer of the present invention is that the composites thereof do not undergo dimensional variations even if in contact with water and saliva during long periods of time.

A further advantage of the resins prepared by polymerization of the acrylic diesters of formula (I) and in particular of the composites which utilize such resins as a matrix, is represented by their very low water absorption. Since, as is known, the absorption of water involves dimensional changes and a faster deterioration of the mechanical and chemical-physical properties of the manufactured article, the products of the present invention posses a chemical inertia much higher than that of the corresponding commercial products.

The invention will be further described with reference to the following illustrative Examples.

Example 1

Into a 10-liter reactor there were introduced 626g of metallyl chloride and 1000g of fluorinated bisphenol A, dissolved in 61g of dimethyl formamide and 392g of potassium hydroxide at 85%.

The reaction mixture was maintained at 30 °C for 8-10 hours. The metallyl ester of fluorinated bisphenol A thus obtained was separated, washed with hexane and water and dried on $Na_2SO_4$. There were obtained 1298g of

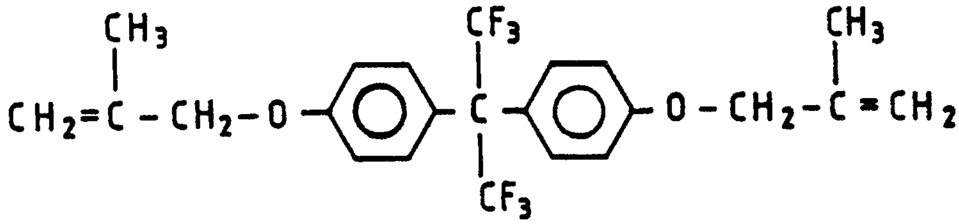

with yield of 90-95%.

The product so obtained was transformed into the corresponding diol through a hydroboration-oxidation reaction. To this purpose, 2332g of $BF_3(C_2H_5)_2O$ were gradually added under stirring to 500g of $NaBH_4$ dissolved in 3.4 1 of diglyme, maintained at 0° C, in a gas-tight reactor. The temperature was brought to 60° C and maintained at such value during 24 hours. The liberated $BH_3$ was absorbed in tetrahydrofuran.

In a gas-tight reactor, the fluorinated dimethallyl-bisphenol A prepared as described hereinbefore was added dropwise to the $BH_3$-tetrahydrofuran solution. The reaction mass was kept at 50° C during 24 hours under continuous stirring.

The organo-borane so obtained was diluted with 1500 ml of water and oxidized with 660 ml of hydrogen peroxide at 35% by volume. The resulting diol was purified on a silica gel column.

1100g of pure diol was reacted with 593g of methacryloylchloride, in the pressence of 872 ml of triethylamine, in 10 l of $CH_2Cl_2$. The esterification reaction was accomplished at -2° C during 1 hour.

The final product was washed with HCl solutions at 5% and NaOH at 40%, and then it was dried.

964g of methacrylic diester were obtained, the yield being 68% referred to the diol.

Example 2

The monomer of Example 1 was polymerized in the presence of 2% of benzoyl peroxide at 80° C for 30 minutes and separately for 5 hours at 110° C. Samples were prepared, measuring 4 cm x 1 cm x 0.5mm, which were immersed into water at 37° C, 48° C and 60° C. Each sample exhibited an absorption of water of about 0.45% by weight.

The samples were subjected to I.R. analyses in order to determine the conversion degree of the methacrylic double bonds. The test was conducted by grinding 100 mg of the sample in liquid nitrogen and by dispersing the resulting powder in KBr.

The absorbance variation of the band at 1639$cm^{-1}$ relating to the double bonds in comparison with the band at 1580 $cm^{-1}$ of the aromatic ring, in the conversion from monomer to polymer, permits the number of reacted double bonds to be calculated. The samples exhibited conversion degrees of 95-96%.

Example 3

By using silanized quartz, the particles of which had average sizes of around 10 µm and a surface area of 1.5 $m^2/g$, there were prepared two pastes consisting of:

A) - 3 g of methacrylic diester of Example 1,
- 7 g of silanized quartz and
- 0.06 g of benzoyl peroxide,

B)- 3 g of methacrylic diester of Example 1,
- 7 g of silanized quartz and
- 0.03 g of dimethyl-paratoluidine.

By mixing 50% by weight of the paste A with 50% by weight of the paste B there were prepared a few samples having dimensions: 4 cm x 1 cm x 0.5 mm.

The samples so obtained, after polymerization at 80° C for 3 minutes, exhibited a water absorption at 37° C, 48° C and 60° C of about 0.15% by weight.

Example 4

By substituting the quartz filler of Example 3 with alumina having a surface area of 4 $m^2/g$, average

particle size of 0.5μm and a narrow particle size distribution, samples were obtained having water absorption of about 0.12%.

The static flexural tests carried out on such samples gave the following results:

- Elastic modulus : 135,000 kg/$cm^2$
- Tensile strength : 980 kg/$cm^2$.

## Claims

1. An acrylic diester of a bisphenol alkyl ether, characterized by having the formula:

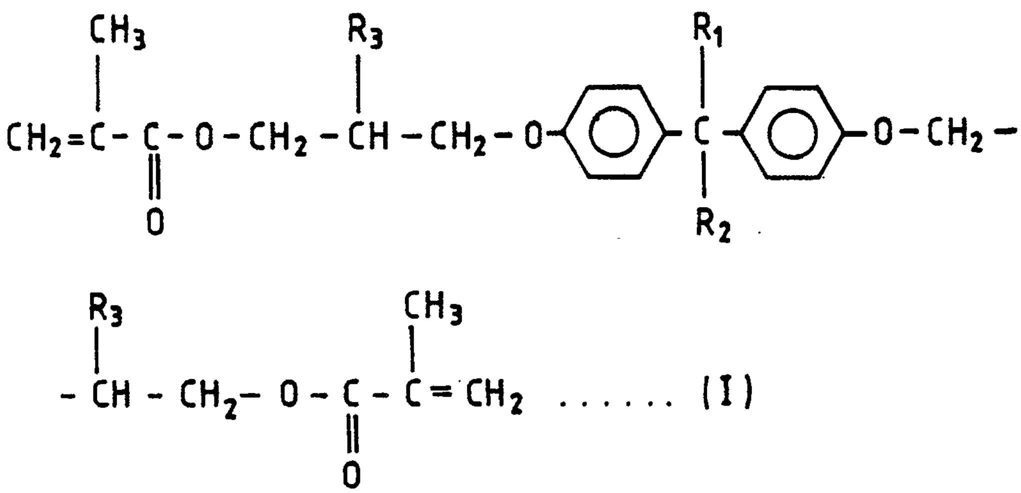

...... (I)

in which $R_1$ and $R_2$ may be the same as or different from each other and are each a linear or ramified alkyl radical containing 1 to 7 carbon atoms substituted by one or more halogens; and $R_3$ may be a hydrogen atom, a halogen atom, or a linear or ramified alkyl radical containing 1 to 7 carbon atoms optionally substituted by one or more halogens.

2. An acrylic diester of bisphenol alkyl ether as claimed in Claim 1, characterized in that $R_1$ and $R_2$ are perfluoroalkyl radicals.

3. An acrylic diester of bisphenol alkyl ether as claimed in Claim 2, characterized in that $R_1$ and $R_2$ are $-CF_3$.

4. An acrylic diester of bisphenol alkyl ether as claimed in any of Claims 1 to 3, characterized in that $R_3$ is an alkyl radical.

5. An acrylic diester of bisphenol alkyl ether as claimed in Claim 4, characterized in that $R_3$ is a methyl radical.

6. An acrylic diester of bisphenol alkyl ether as claimed in any of Claims 1 to 5, characterized in that one or more hydrogen atoms of the two benzene rings is/are substituted by an alkyl radical or an alkoxy radical containing 1 to 4 carbon atoms.

7. An acrylic resin characterized by containing repeating units of an acrylic diester of a bisphenol alkyl ether of formula:

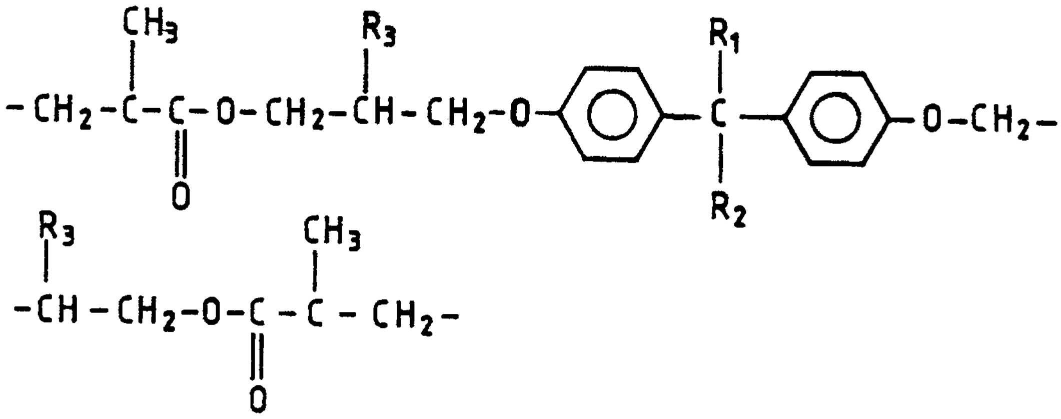

in which $R_1$ and $R_2$ may be the same or different from each other and are each a linear or ramified alkyl radical containing 1 to 7 carbon atoms substituted by one or more halogens; and $R_3$ may be a hydrogen atom, a halogen atom, or a linear or ramified alkyl radical containing 1 to 7 carbon atoms optionally substituted by one or more halogens.

**8.** An acrylic resin as claimed in Claim 7, characterized in that $R_1$ and $R_2$ are perfluoro-alkyl radicals, preferably $-CF_3$.

**9.** An acrylic resin as claimed in Claim 7 or 8, characterized in that $R_3$ is a methyl radical.

**10.** An acrylic resin as claimed in any of Claims 7 to 9, characterized in that one or more hydrogen atoms of the two benzene rings is/are substituted by an alkyl radical or an alkoxy radical containing 1 to 4 carbon atoms.

**11.** A dental composite characterized by comprising an acrylic diester of a bisphenol alkyl ether having formula (I) as claimed in any of Claims 1 to 6, at least one inorganic filler, and a catalytic amount of a catalytic redox system.

**12.** A dental composite as claimed in Claim 11, characterized in that the inorganic filler is present in an amount of from 30 to 85% by weight.

**13.** A dental composite as claimed in Claim 12, characterized in that the inorganic filler is present in an amount of from 50 to 80% by weight.

**14.** A dental composite as claimed in any of Claims 11 to 13, characterized in that the inorganic filler is selected from quartzes, silicas, Al, Ba and Sr silicates, zirconates, and aluminas having a low surface area, a diameter of the particles below 40 $\mu$m, and treated with silanes, glass fibres or carbon fibres, and submicronic inorganic fillers, brought to sizes of the order of 10-40 $\mu$m and coated with methacrylates.

**15.** A dental composite as claimed in any of Claims 11 to 14, characterized in that the amount of the catalytic redox system is from 0.05 to 5% by weight referred to the monomer.

**16.** A dental composite as claimed in any of Claims 11 to 15, characterized by containing a diluent.

**17.** A method of preparing an acrylic diester of bisphenol alkyl ether according to any of Claims 1 to 6, characterized by taking a diol of halogenated p,p'-dihydroxy-diphenyl alkane, and

a) directly esterifying said diol with acrylic or methacrylic acid in the presence of an esterification catalyst; or

b) trans-esterifying an alkyl ester of acrylic or methacrylic acid with said diol in the presence of an acid or a basic catalyst; or

c) reacting said diol with a reactive derivative of acrylic or methacrylic acid, such as chloride or

anhydride.

18. A method of preparing an acrylic resin, characterized by polymerizing an acrylic diester of bisphenol alkyl ether as claimed in any of Claims 1 to 6 or a said acrylic diester obtained by the method as claimed in Claim 17.

19. The use of an acrylic diester of bisphenol alkyl ether as claimed in any of Claims 1 to 6 or a said acrylic diester obtained by the method as claimed in Claim 17, together with at least one inorganic filler and a catalytic amount of a catalytic redox system, in the preparation of a dental composite.

## Revendications

1. Un diester acrylique d'éther alkylique de bisphénol, caractérisé par la formule

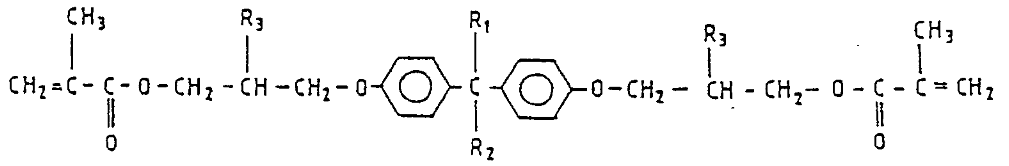
...... (I)

dans laquelle R1 et R2 peuvent être identiques ou différents l'un de l'autre et représentent chacun un radical alkyle linéaire ou ramifié, contenant de 1 à 7 atomes de carbone, substitué par un ou plusieurs atomes d'halogène; et R3 peut représenter un atome d'hydrogène, un atome d'halogène ou un radical alkyle linéaire ou ramifié contenant de 1 à 7 atomes de carbone éventuellement substitué par un ou plusieurs atomes d'halogène.

2. Un diester acrylique d'éther alkylique de bisphénol selon la revendication 1, caractérisé en ce que R1 et R2 sont des radicaux perfluoroalkyles.

3. Un diester acrylique d'éther alkylique de bisphénol selon la revendication 2, caractérisé en ce que R1 et R2 représentent -CF3.

4. Un diester acrylique d'éther alkylique de bisphénol selon l'une quelconque des revendications 1 à 3, caractérisé en ce que R3 représente un radical alkyle.

5. Un diester acrylique d'éther alkylique de bisphénol selon la revendication 4, caractérisé en ce que R3 est un radical méthyle.

6. Un diester acrylique d'éther alkylique de bisphénol selon l'une quelconque des revendications 1 à 5, caractérisé en ce qu'un ou plusieurs atomes d'hydrogène des deux cycles benzéniques sont substitués par un radical alkyle ou par un radical alcoxy contenant de 1 à 4 atomes de carbone.

7. Une résine acrylique, caractérisée en ce qu'elle contient des motifs répétitifs d'un diester acrylique d'un éther alkylique de bisphénol de formule

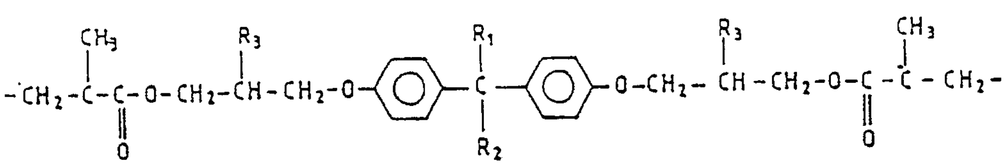

dans laquelle R1 et R2 peuvent être identiques ou différents l'un de l'autre et représentent chacun un radical alkyle linéaire ou ramifié contenant de 1 à 7 atomes de carbone substitué par un ou plusieurs atomes d'halogène; et R3 peut représenter un atome d'hydrogène, un atome d'halogène ou un radical alkyle linéaire ou ramifié contenant de 1 à 7 atomes de carbone, éventuellement substitué par un ou plusieurs atomes d'halogène.

8. Une résine acrylique selon la revendication 7, caractérisée en ce que R1 et R2 sont des radicaux perfluoroalkyles, de préférence -CF3.

9. Une résine acrylique selon la revendication 7 ou 8, caractérisée en ce que R3 est un radical méthyle.

10. Une résine acrylique selon l'une quelconque des revendications 7 à 9, caractérisée en ce qu'un ou plusieurs atomes d'hydrogène des deux cycles benzéniques sont substitués par un radical alkyle ou un radical alcoxy contenant de 1 à 4 atomes de carbone.

11. Un matériau dentaire composite, caractérisé en ce qu'il comprend un diester acrylique d'éther alkylique de bisphénol répondant à la formule (I) selon l'une quelconque des revendications 1 à 6, au moins une charge inorganique et une quantité catalytique d'un système redox catalytique.

12. Un matériau dentaire composite selon la revendication 11, caractérisé en ce que la charge inorganique est présente en quantité de 30 à 85% en poids.

13. Un matériau dentaire composite selon la revendication 12, caractérisé en ce que la charge inorganique est présente en quantité de 50 à 80% en poids.

14. Un matériau dentaire composite selon l'une quelconque des revendications 11 à 13, caractérisé en ce que la charge inorganique est choisie parmi quartz, silices, silicates de Al, Ba et Sr, zirconates et alumines présentant une faible surface spécifique, présentant un diamètre de particules inférieur à 40 µm, et traités à l'aide de silanes, de fibres de verre ou de fibres de carbone et des charges minérales submicroniques portées aux dimensions de l'ordre de 10 à 40 µm et revêtues de méthacrylates.

15. Un matériau dentaire composite selon l'une quelconque des revendications 11 à 13, caractérisé en ce que la quantité de système redox catalytique est comprise entre 0,05 et 5% en poids par rapport au monomère.

16. Un matériau dentaire composite selon l'une quelconque des revendications 11 à 15, caractérisé en ce qu'il contient un diluant.

17. Un procédé de préparation d'un diester acrylique d'éther alkylique de bisphénol selon l'une quelconque des revendications 1 à 6, caractérisé en ce que l'on prend un diol de p,p'-dihydroxy-diphényl alcane halogéné et
   a) on estérifie directement ce diol à l'aide d'acide acrylique ou d'acide méthacrylique en présence d'un catalyseur d'estérification; ou
   b) on procède à une transestérification d'un ester alkylique d'acide acrylique ou d'acide méthacrylique par ce diol en présence d'un catalyseur acide ou d'un catalyseur basique; ou
   c) on fait réagir ce diol avec un dérivé réactif d'acide acrylique ou d'acide méthacrylique tel que leur chlorure ou leur anhydride.

18. Un procédé de préparation d'une résine acrylique, caractérisé en ce que l'on polymérise un diester acrylique d'éther alkylique de bisphénol selon l'une quelconque des revendications 1 à 6, ou ce diester acrylique obtenu par le procédé selon la revendication 17.

19. L'utilisation pour la préparation d'un matériau dentaire composite d'un diester acrylique d'éther alkylique de bisphénol selon l'une quelconque des revendications 1 à 6 ou du diester acrylique obtenu par le procédé selon la revendication 17, en même temps qu'au moins une charge minérale et une quantité catalytique d'un système redox.

**Patentansprüche**

1. Acrylsäurediester eines Bisphenolalkyläthers, gekennzeichnet durch die folgende Formel:

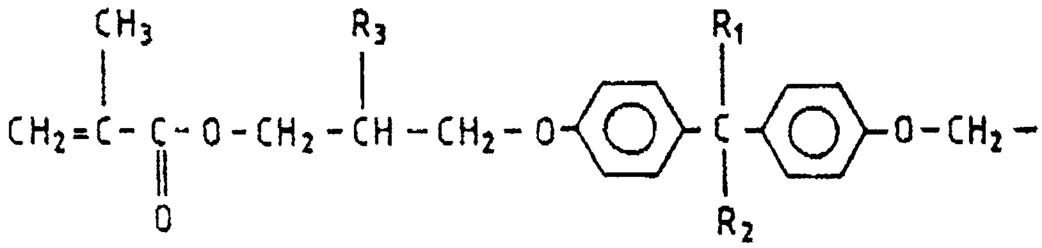

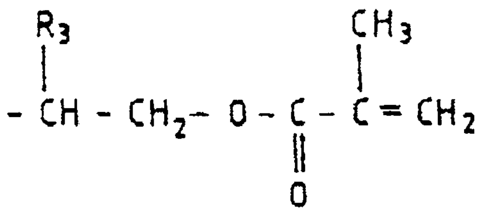 ...... (I)

in welcher $R_1$ und $R_2$, die gleich oder voneinander verschieden sein können, jeweils die Bedeutung eines linearen oder verzweigten Alkylrestes mit 1 bis 7 Kohlenstoffatomen haben, die mit einem oder mehreren Halogenen substituiert sind, und in welcher $R_3$ ein Wasserstoffatom, ein Halogenatom oder ein linearer oder verzweigter Alkylrest mit 1 bis 7 Kohlenstoffatomen sein kann, der wahlweise mit einem oder mehreren Halogenen substituiert ist.

**2.** Acrylsäurediester eines Bisphenolalkyläthers nach Anspruch 1, dadurch gekennzeichnet, daß $R_1$ und $R_2$ Perfluoralkylreste sind.

**3.** Acrylsäurediester eines Bisphenolalkyläthers nach Anspruch 2, dadurch gekennzeichnet, daß $R_1$ und $R_2$ $-CF_3$ sind.

**4.** Acrylsäurediester eines Bisphenolalkyläthers nach irgendeinem der Ansprüche 1 bis 3, dadurch gekennzeichnet, daß $R_3$ ein Alkylrest ist.

**5.** Acrylsäurediester eines Bisphenolalkyläthers nach Anspruch 4, dadurch gekennzeichnet, daß $R_3$ ein Methylrest ist.

**6.** Acrylsäurediester eines Bisphenolalkyläthers nach irgendeinem der Anpsürche 2 bis 5, dadurch gekennzeichnet, daß ein oder mehrere Wasserstoffatom(e) der beiden Benzolringe durch einen Alkylrest oder einen Alkoxyrest mit 1 bis 4 Kohlenstoffatomen substituiert ist/sind.

**7.** Acrylharz, dadurch gekennzeichnet, daß es sich wiederholende Einheiten eines Acrylsäurediesters eines Bisphenolalkyläthers der Formel:

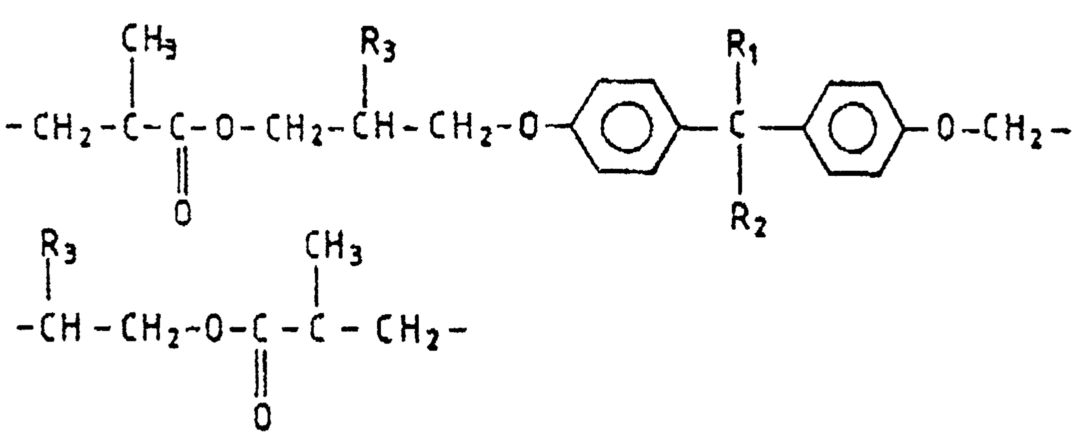

enthält, in welcher $R_1$ und $R_2$, die gleich oder voneinander verschieden sein können, jeweils die

Bedeutung eines linearen oder verzweigten Alkylrestes mit 1 bis 7 Kohlenstoffatomen haben, die mit einem oder mehreren Halogenen substituiert sind; und in welcher $R_3$ ein Wasserstoffatom, ein Halogenatom oder ein linearer oder verzweigter Alkylrest mit 1 bis 7 Kohlenstoffatomen sein kann, der wahlweise mit einem oder mehreren Halogenen substituiert ist.

**8.** Acrylharz nach Anspruch 7, dadurch gekennzeichnet, daß $R_1$ und $R_2$ Perfluoralkylreste sind, vorzugsweise $-CF_3$.

**9.** Acrylharz nach den Ansprücher 7 oder 8, dadurch gekennzeichnet, daß $R_3$ ein Methylrest ist.

**10.** Acrylharz nach irgendeinem der Ansprüche 7 bis 9, dadurch gekennzeichnet, daß ein oder mehrere Wasserstoffatom(e) der beiden Benzolringe durch einen Alkylrest oder einen Alkoxyrest mit 1-4 Kohlenstoffatomen substituiert ist/ sind.

**11.** Zusammensetzung für den zahnärztlichen Gebrauch, dadurch gekennzeichnet, daß sie einen Acrylsäurediester eines Bisphenolalkyläthers der Formal (I) nach irgendeinem der Ansprüche 1 bis 6, mindestens einen anorganischen Füllstoff und eine katalytische Menge eines katalytischen Redoxsystems umfaßt.

**12.** Zusammensetzung für den zahnärztlichen Gebrauch nach Anspruch 11, dadurch gekennzeichnet, daß der anorganische Füllstoff in einer Menge von 30 bis 85 Gew.-% vorliegt.

**13.** Zusammensetzung für den zahnärztlichen Gebrauch nach Anspruch 12, dadurch gekennzeichnet, daß der anorganische Füllstoff in einer Menge von 50 bis 80 Gew.-% vorliegt.

**14.** Zusammensetzung für den zahnärztlichen Gebrauch nach irgendeinem der Ansprüche 11 bis 13, dadurch gekennzeichnet, daß der anorganische Füllstoff ausgewählt ist aus: Quarzen, Siliciumdioxid, Al, Ba und Sr Silikaten, Zirkonaten und Aluminiumoxiden, die eine kleine Oberfläche besitzen, einen Partikeldurchmesser von weniger als 40 $\mu m$ haben und die mit Silanen behandelt sind, aus Glasfasern oder Kohlenstofffasern und submikroskopischen anorganischen Füllstoffen, die auf Größen der Größenordnung von 10 bis 40 $\mu m$ gebracht sind und mit Methacrylaten überzogen sind.

**15.** Zusammensetzung für den zahnärztlichen Gebrauch nach irgendeinem der Ansprüche 11 bis 14, dadurch gekennzeichnet, daß die Menge des katalytischen Redoxsystems (auf das Monomer bewogen) von 0,05 bis 5 Gew.-% beträgt.

**16.** Zusammensetzung für den zahnärztlichen Gebrauch nach irgendeinem der Ansprüche 11 bis 15, dadurch gekennzeichnet, daß sie ein Verdünnungsmittel enthält.

**17.** Verfahren zur Herstellung eines Acrylsäurediesters eines Bisphenolalkyläthers nach irgendeinem der Ansprüche 1 bis 6, dadurch gekennzeichnet, daß von einem Diol eines halogenierten p,p'-Dihydroxydiphenylalkan ausgegangen wird, und daß

a) dieses Diol in der Gegenwart eines Veresterungskatalysators direkt mit Acryl- oder Methacrylsäure verestert wird; oder daß

b) ein Alkylester der Acryl- oder Methacrylsäure mit diesem Diol in der Gegenwart einer Säure oder eines basischen Katalysators umgeestert wird; oder daß

c) dieses Diol mit einem reaktiven Derivat der Acryl-oder Methacrylsäure, wie z.B. einem Chlorid oder Anhydrid, umgesetzt wird.

**18.** Verfahren zur Herstellung eines Acrylharzes, dadurch gekennzeichnet, daß ein Acrylsäurediester eines Bisphenolalkyläthers nach irgendeinem der Ansprüche 1 bis 6 oder ein solcher Acrylsäurediester, wie er nach dem Verfahren gemäß Anspruch 17 erhalten wird, polymerisiert wird.

**19.** Verwendung eines Acrylsäurediesters eines Bisphenolalkyläthers gemäß irgendeinem der Ansprüche 1 bis 6 oder eines solchen Acrylsäurediesters, wie er nach dem Verfahren gemaß Anspruch 17 erhalten wird, zusammen mit mindestens einem anorgnischen Füllstoff und einer katalytischen Menge eines katalytischen Redoxsystems, zur Herstellung einer Zusammensetzung für den zahnärstlichen Gebrauch.